# EUROPEAN PATENT SPECIFICATION

(11) **EP 2 376 692 B1**
(45) Date of publication and mention of the grant of the patent: **10.07.2013**
(21) Application number: 10701125.6
(22) Date of filing: 13.01.2010
(51) Int. Cl.: D04H 1/56, D01D 5/098

(54) **BIOMIMETIC NANOFIBER WEB AND METHOD AND DEVICE TO MANUFACTURE THE SAME**
BIOMIMETISCHE NANOFASERBAHN SOWIE VERFAHREN UND VORRICHTUNG ZU IHRER HERSTELLUNG
NAPPE DE NANOFIBRES BIOMIMÉTIQUES, PROCÉDÉ ET DISPOSITIF DE FABRICATION ASSOCIÉS

(30) Priority: 13.01.2009 EP 09305033
(43) Date of publication of application: 19.10.2011
(73) Proprietor: INSERM - Institut National de la Santé et de la Recherche Médicale, 75013 Paris (FR); Universite de Nantes, 44000 Nantes (FR)
(72) Inventor: SOHIER, Jérome, F-44000 Nantes (FR); LAYROLLE, Pierre, F-44000 Nantes (FR)
(74) Representative: Cabinet Plasseraud
(86) International application number: PCT/EP2010/050359
(87) International publication number: WO 2010/081832

(56) References cited:
- WO-A-2006/113791
- DE-A1-102005 050 560
- US-A1- 2002 148 050
- US-A1- 2005 249 654

## Description

### Field of the invention

The invention relates to the field of biomimetic nanofiber web and method and device to manufacture it.

### Background of the invention

Extra cellular matrix (ECM) is a complex and dynamic environment secreted by the cells to provide the structure of a tissue. Aside from proteoglycans, the main component of ECM is fibrillar collagen that serves as mechanical framework, anchor point to the cells and provides regulatory signals. In an organism, tissues are constructed with a bottom-up approach where the cells first synthesize an optimal extracellular matrix that in turn supports cell proliferation and the resulting defined tissue organization.

Therefore, it will be particularly useful to provide a defined artificial fibrillar matrix to cells so that they organize in a new tissue accordingly, for instance for bone reconstruction or arteries replacement.

The use of natural collagen is hampered by low availability from allogenic sources and the possibility to transfer pathogens from xenogenic sources. Optimally, the synthetic mimetic scaffold should provide the cells with a structure similar to native collagen networks in its organization and properties.

Current approaches to fabricate fibers with size comparable to collagen fibers mainly include self assembly, phase separation and electrospinning. Notably, although the first two techniques can shape polymer nanofibers, they have limited control over fiber diameter, orientations and continuity.

Electrospinning is the most employed method as it provides a continuous formation of fibers in the micrometer to hundreds of nanometer range that revealed promising for different tissue engineering applications.

Briefly, electro-spinning consists in applying a high tension between a viscous polymer solution and a collector. The polymer solution is extruded at a defined feed rate through a nozzle and gets charged. Once this charge exceeds the viscoelastic force and surface tension of the polymer, a polymer jet is ejected from the nozzle towards the oppositely charged collector. In transit, the solvent evaporates and solid fibers are therefore collected. By varying the different preparation parameters (polymer concentration, flow rate or electric tension) this method allows to create nanofibrous structures of defined fiber diameters and orientation that were promising for different tissue engineering applications. Murugan R. et al. "Design strategies of tissue engineering scaffolds with controlled fiber orientation", Tissue Eng., 2007.13(8), pp. 1845-66 and US 6 924 028 discuss thoroughly electro-spinning. However this method requires a very high electric tension in the range of tens of kilovolts to produce fibers. Although the electric field current is usually low, such a high voltage can be dangerous for the manipulator. Furthermore, the up-scaling of this process for industrial applications might reveal to be problematic.

Other methods such as, for instance, those described by W02006/113791, US 6 315 806, WO2007/12145 and US 6 382 526 documents use molded polymeric material projected onto a surface or flashed by a abrupt decrease of pressure ("flash spinning") as disclosed by US 1 211 737, US 4 081 226, US 5 032 326. However, the use of molded polymer means that high temperature is required. With this constraint, the method is limited to nanofiber web comprising only one type of nanofiber/component.

### Summary of the invention

It would advantageous to achieve a method which is easy to set up, without undue constraint in term of safety or parameters in order to produce low cost and high quality nanofiber web.

To better address one or more concerns, in a first aspect of the invention a method for forming nanofiber web comprising the steps of:
● mixing polymeric material with a solvent to obtain a mixture having a viscosity above a predetermined shear viscosity value at room temperature;
● feeding a spray nozzle with said mixture;
● projecting said mixture through said nozzle with a gas jet, wherein the projected polymeric material solidifies and forms nanofibers;
● depositing said nanofibers on a collecting surface to form said nanofiber web.

The use of a polymer/solvent mixture allows advantageously nanofiber web manufacturing at ambient temperature and without potentially hazardous electrical field. Furthermore, the method achieves a good control of nanofiber geometry.

In particular embodiments:
● the said predetermined shear viscosity value is equal to 0.068 Pa.s.for a shear rate increasing from 0.05 to 9000 s⁻¹ in 60s;
● the shear viscosity of said mixture is above 0.1 Pa.s. for a shear rate increasing from 0.05 to 9000 s⁻¹ in 60s;
● the mixture comprises biological active molecules;
● the spray nozzle comprises a passageway for directing said mixture outside, said passageway being partially obstructed by an internal needle parallel to the mixture flow, said needle diffracting the mixture outflow;
● the spray nozzle is of internal or external mixing type;
● the gas jet sucks up the mixture outside the spray nozzle;
● the mixture is at ambient temperature;
● the polymeric material comprises any solvent-soluble polymer, particularly polyester.

In a second aspect of the invention, a device for forming nanofiber web comprises:
● a container of a mixture of a polymeric material and a solvent at room temperature, said mixture having a shear viscosity above a predetermined viscosity value, the container being connected to
● a spray nozzle fed with said mixture;
● a gas jet inlet, connected to the spray nozzle so that the gas jet projects said mixture through said nozzle and the projected polymeric material solidifies and forms nanofibers;
● a collecting surface on which said nanofibers deposit to form said nanofiber web.

In a particular embodiment, the collecting surface is formed by the surface of a liquid in which the nanofibers are not soluble.

In a third aspect of the invention, biomimetic nanofiber web is produced by the preceding method and the average mesh size of interlaced polymeric nanofibers is greater than 10µm and the average diameter of the nanofibers is less than 800 nm.

In particular embodiments, the biomimetic nanofiber web further comprises biological active molecules interlaced between the polymeric nanofibers, and the biological active molecules may be made of calcium phosphate.

These and other aspects of the invention will be apparent from and elucidated with reference to the embodiment described hereafter where:
- Figure 1 is a schematic view of a device to make nanofiber web according to one embodiment of the invention;
- Figure 2 is a schematic view of a method according to an embodiment of the invention;
- Figure 3 is a diagram of nanofiber diameter distribution when the device of Figure 1 is used with two different sets of parameters;
- Figure 4 is a diagram of fluorescent signal function of time for a cell culture treated plastic of the prior art and a nanofiber web produced by the device of Figure 1; and
- Figure 5 is a schematic view of a device according to a second embodiment of the invention.

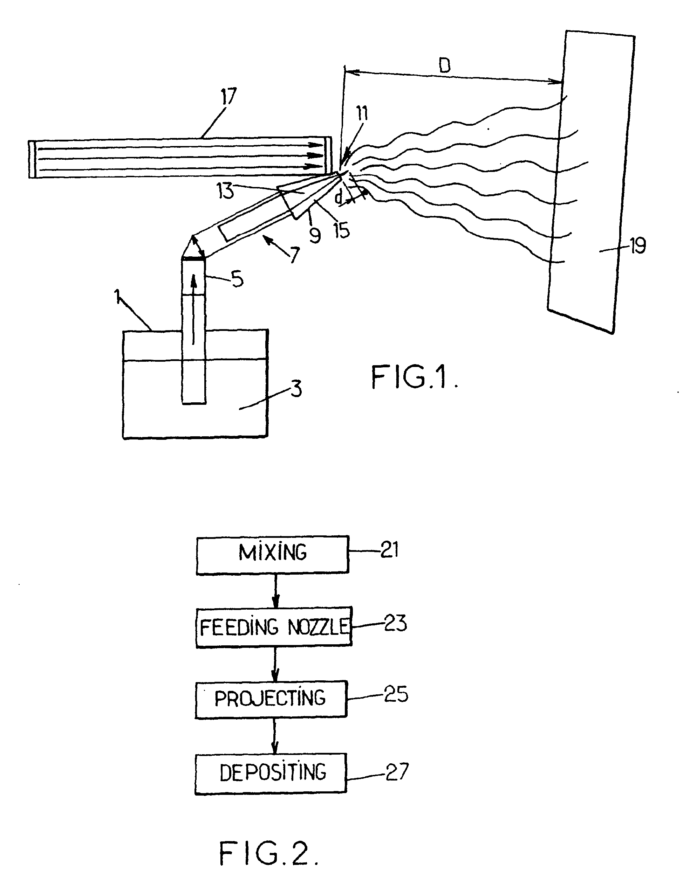

In reference to Figure 1, a container 1 contains a mixture 3 of polymeric material and solvent. The ratio of polymeric material and solvent is such that the mixture 3 has a viscosity above a predetermined viscosity value.

A pipe 5 connects the container 1 to a nozzle 7 so that the mixture 3 feeds the nozzle 7.

The nozzle 7 comprises an outer conical tube 9 having an outlet orifice 11. A center conical needle 13 is movable longitudinally along its axis. A passageway 15 is defined between the outer tube 9 and the needle 13. The mixture 3 is transferred toward the outlet orifice 11 through the passageway 15. The conical aspect of the outer tube 9 and the needle 13 is such that the needle 13, by moving longitudinally, adjusts the size of the outlet orifice 11.

The outlet orifice size may be defined by the distance d between the needle tip and the outer tube end, where d is positive, by convention, when the needle summit is inside the outer tube 9.

A tube 17 conducts a gas jet towards the external of the outlet orifice 11.

A collecting surface 19 is disposed at a distance D of the outlet orifice 11.

As illustrated, the tube 17 and the nozzle 7 form an acute angle such that the gas jet creates a depression at the outlet orifice 11.

The gas jet is typically compressed air at a pressure of 3 to 6 bars.

By creating the depression, the gas jet drives the polymer mixture from the container 1 to the nozzle 3 where it is diffracted by the needle 13 and projected to the collecting surface 19.

Upon projection, the polymer solvent is rapidly evaporated and the polymer collected as non woven fibers on the collecting surface 19.

On transit, the cohesion of the polymer phase and the solvent evaporation allows the formation of smooth, non woven fibers that are collected as homogeneous mats of various and complex shapes. The fibers, of smooth surface and ranging from hundreds of nm to few µm, are interconnected by physical entanglements and contact adhesion and form an open network or web.

The collecting surface 19 is either a static metallic grid to create nanofibrous mats or a rotating mandrel to form a non-woven tubular structure.

Furthermore, the ability to move the spraying device in any direction and at any angle allowed covering targets of complex shapes with nanofibrous networks.

The collecting surface 19 may also be a liquid in which the polymer is not soluble such as water, ethanol, etc. The nanometer sized fibers are thus deposited onto the surface of the liquid and sink. The resulting structures are open meshes or foams made of nanometer sized polymer fibers.

A dense polymer film may be deposited on the collecting surface by dip coating before or after applying the nanofibers. The resulting membrane is thus composed of a dense film sandwiched by open mesh of nanofibers.

Thus, the method of forming nanofiber web comprises, Figure 2, the steps of:
● Mixing, step 21, polymeric material with a solvent to obtain a mixture having a viscosity above a predetermined viscosity value;
● Feeding, step, 23, a spray nozzle with the mixture;
● Projecting, step 25, the mixture through the nozzle with a gas jet such that the projected polymeric material solidifies and forms nanofibers;
● Depositing, step 27, the nanofibers on a collecting surface to form the nanofiber web.

As an example, a detailed experiment is disclosed hereafter.

Poly (ε-Carpolactone) (PCL, 80000 g/mol) was selected for its biocompatibility and mechanical properties, slow degradation rate and ability of supporting a wide variety of cell types. The polymer mixture is created by adding, as solvent, chloroform starting from a concentration of 0,06 g of polymer per ml of solvent, giving a shear viscosity of around 0.068 Pa.s. The viscosity is measured at room temperature (25°C) by a cone and plane viscometer using a dynamic shear rate increasing from 0.05 to 9000 1/s in 60 seconds.

Upon spraying, the polymer strain combined with solvent evaporation resulted in solid and smooth fibers ranging from hundreds of nanometers to few micrometers. These fibers were interconnected in an open network by physical entanglements and contact adhesion.

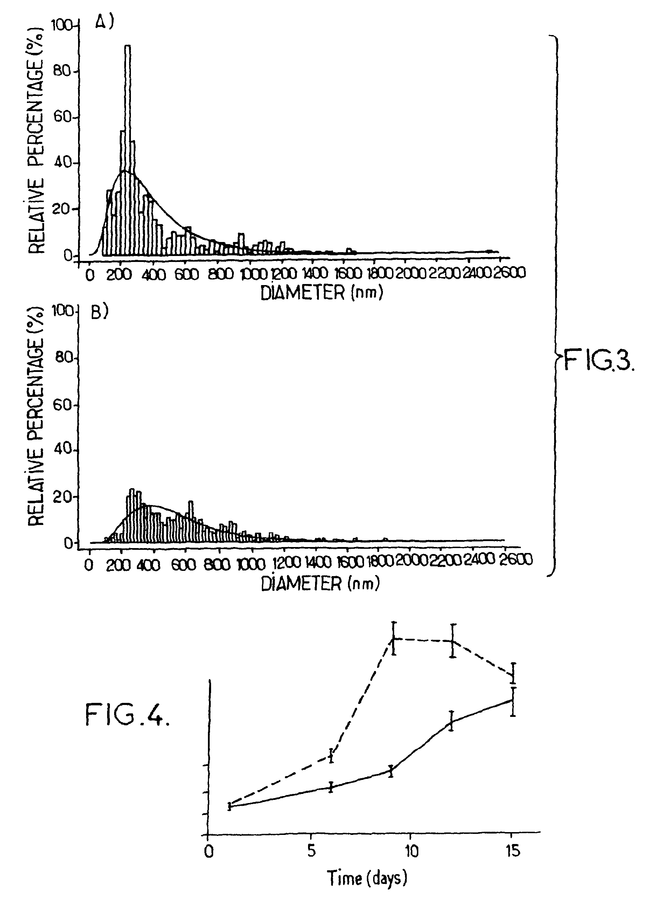

Similar results with regards to the formation of nanofibers are obtained with different polymers such as poly lactic-co-glycolic acid and poly lactic acid. The fibers diameter followed, Figure 3, a lognormal distribution characterized by an asymmetric shape and tails at the large diameter end. The distribution width and the average diameter are not correlated.

In Figure 3, the diagram A illustrates the fiber diameter distribution obtained with a polymer concentration of 1 g/15ml, an open nozzle (d = 1 mm), an air pressure of 6 10⁵ N/m² (bar) and a distance D of 25cm. The diagram B illustrates the fiber diameter distribution obtained with a polymer concentration of 1g/10ml, an almost close nozzle (d = -1 mm), an air pressure of 3 10⁵ N/m² (bar) and a distance D of 35cm.

To investigate the effect of processing parameters on fiber distribution, a full 2-level factorial design (randomized 24 runs with 4 random center points) was conducted. Four easily adjustable parameters were considered as variables in the factorial design: polymer concentration (0.06 - 0.1 g/ml), nozzle opening (distance needle tip-nozzle -1 - 1 mm), air pressure (3 - 6 bars) and spraying distance (25 - 35 cm). Several hundreds diameters were measured for each sample and statistically analyzed with an analysis of variance. Average fiber diameter (adjusted between 300 and 600 nm) are controlled by the polymer concentration (p=0.0004) and the nozzle opening (p= 0.01). On the whole, increasing polymer concentrations leads to bigger fibers while opening the nozzle results in smaller ones. The population homogeneity, given by the distribution span (intercentile 1-99%), could be tailored from 600 nm (100-700 nm) to 1900 nm (100-2000 nm) mainly by increasing the spraying distance (p=0.04). The effect of distance can be related to the whipping of the polymer fibers within the airflow that produces an extensional force and elongates the fibers. Longer spraying distances provide more time for higher elongations and therefore more homogeneous distributions. Conversely, the effect of polymer concentration on fibers diameter can be easily linked to the solution viscosity. Indeed, viscous solutions oppose more resistance to elongation forces. Overall, within the limits set in the factorial design, polymer spraying allowed to construct nanofibers of controlled average diameters that range from 300 to 600 nm. It is worth noting that although electrospinning can theoretically produce nanofibers of comparable diameters, they are seldom reported in the literature. Electrospun fibers seem to be in majority above the micrometer scale. The disclosed spraying technique could also produce fibers with average diameter 800 nm and 1.1 µm by increasing the concentration of the polymer solutions respectively to 0.12 and 0.15 g/ml.

To investigate cellular response to fibers closely mimicking collagen (c.a. 500 nm), human mesenchymal stromal cells (hMSC, 5.105) isolated from the marrow aspirate of a young female patient were plated on top of nanofibrous mats and cultured statically. As an indication that the nanofibrous structures had been recognized, the cells attached, acquired a fibroblastic phenotype within a few hours and spread to cover the surface. Entangled fibers within the scaffolds did not obstruct cell-invasion, which attained 500 to 600 µm after 21 days of culture. Cellular penetration is an essential parameter to provide a functional tissue. Unlike electrospun structures that very often result in a monolayer of cell on their surface when cultured, statically sprayed nanofibers were infiltrated efficiently.

Cellular proliferation within the structures was examined and compared, Figure 3, to culture-treated plastic. The plain line shows the fluorescent signal intensity of a nanofiber web and the dotted line shows the same for the plastic as a function of time. The synthetic nanofibrillar network supported hMSCs proliferation, suggesting that the cells penetrated inside the nanofibrous structures partly by cellular division. In addition to proliferation and migration within the sprayed structures, hBMCs remodeled their local environment by producing collagen 1, as evidenced by immunohistochemical staining. The sprayed nanofibers therefore provided a suitable template for tissue formation as the microfiber web formed by these interlaced nanofibers as an average mesh size greater than 10µm. With this size, human mesenchymal cells, such as fibroblasts, which have a sectional size comprises between 5 and 10µm can migrate inside the web.

The ability of the nanofibrillar matrices to support osteogenic differentiation of hMSCs was investigated in an osteogenic medium (10 mM (millimole / litre) beta-glycerophosphate, 0.2 mM ascorbic acid-2-phosphate and 10-8 M dexamethasone). Cells expressed alkaline phosphatase activity after 11 days and significant matrix calcification was observed after 21 days, showing osteoblastic differentiation of the stromal cells within the nanofibrillar matrices. Immunohistochemistry staining revealed the production of osteoblastic markers (bone sialo protein and osteopontin). Even more interesting for bone regeneration and engineering, the nanofibrillar matrix supported the formation of micro-crystals that resemble apatite, both between and on the nanofibers. The crystals were effectively composed of calcium and phosphorus, like bone apatite. These findings suggest that the cells recognized the sprayed structures as a suitable three-dimensional template for producing their own collagenous ECM that they mineralized in vitro.

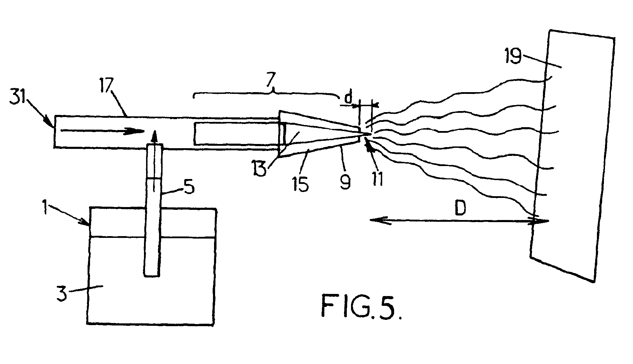

Figure 5 illustrates another embodiment of the spraying device. Similar features are referenced by same numbers. The main difference is in the nozzle construction in which the gas jet is introduced by a gas inlet 31 into the nozzle passageway 15 so that the polymer mixture and the gas are mixed in before being ejected through the outlet orifice 11.

Generally speaking, any type of spray nozzle may be used either of external mixing type, i.e. the gas and the mixture are mixed outside the nozzle, either of internal mixing type, i.e. the gas and the mixture are mixed inside the nozzle (see article "Spray Nozzle" in Wikipedia). Based on the disclosed teaching of this document, the man skilled in the art is able to define the parameters to achieve nanofiber web with desired features.

A particularly useful feature of the described device and method is that the viscosity of the polymer solution is achieved by mixing it into a solvent and not by heating the solution. Thus, it is possible to add some biological active molecules dissolved in aqueous solution forming a micro emulsion with the mixture of polymer and solvent, some of them being destroyed by the melting temperature otherwise. For instance, growth factors such as bone morphogenetic proteins, basic fibroblast growth factor, transforming growth factor, growth hormone or glucocorticoids like dexamethasone may be incorporated into the nanofibrillar structures. In order to prevent post surgical infections, it may be desired to incorporate antibiotics in the nanometer sized polymer fibers.

The disclosed method and devices produce films or membranes made of nanometer sized fibers of any polymer. The organic solvent is preferably chloroform, dichloromethane, and methanol. The polymer is generally chosen to be biodegradable in the human body and is composed of poly(α-hydroxy esters), poly(ε-caprolactone), poly(dioxanone), poly(ortho esters), poly(amide esters), poly(anhydrides) or polyvinyl esters. However, for some medical applications, the polymer may also be non degradable or mixtures with various degrees of biodegradation, as (poly(tetrafluoroethylene), poly(ethylene), poly(ethylene glycol),poly(propylene oxide).

The films or membranes produced by the method described herein are composed of nanometer fibers of polymers. The thickness of polymer membrane may vary between 0.05 to 100 mm. Depending on the porosity and/or network of nanometer sized fibers, the resulting mesh may allow body fluid infiltration, cell colonization and tissue in growth as well as vascularization. The synthetic membranes produced by the disclosed method and device are used in guided tissue regeneration. In dental surgery, polymer-calcium phosphate composite membranes may be prepared by using two settings simultaneously operated. One spraying device is used for depositing biodegradable polymer nanometer sized fibers into a film while another device sprays a calcium phosphate powder onto the nanofiber web. The two compounds may also be spray simultaneously in the same device using an emulsion of organic solvent and water. The aqueous solution may contain calcium and phosphate salts that would precipitate onto the nanometer sized fiber mesh. The calcium phosphate compounds are bioactive and osteoconductive thus guiding bone regeneration while the polymer membrane prevent fibrosis and gingival tissue in growth in the alveolar bone. Such synthetic composite membranes may be used also for bone augmentation, crest elevation, sinus lift, etc prior to dental implants. In these applications, the membranes are suturable and shapeable. These synthetic membranes are superior to collagen or other animal or human derived membranes currently in used as they do not transfer diseases or immunological adverse reactions, and their mechanical and degradability in the body are easily controlled.

The synthetic membranes are also usable for gastro intestinal surgery. Such membranes may prevent or treat post surgical adherences, be used for closing peritonea membrane or for treating inguinal hernia. As previously indicated, the degradability in the human body and mechanical properties of such membranes may be adjusted by the chemical nature of the chosen polymer and by the thickness of membranes. It is also possible to deposit two, or more, polymers simultaneously with different mechanical and biodegradation rates by using simultaneously one spraying device per polymer to be mixed or by preparing a mixture containing the different polymers in the desired ratio for spraying.

Another important medical application of the biomimetic nanometer sized polymer webs is cardiovascular surgery. Indeed, artificial vascular grafts with diameter under 5 mm are not available because most of polymer tubes are subjected to blood clotting and thrombosis. It is possible by using the present invention to produce small diameter polymer tubes that may be colonized by smooth muscle and endothelial cells thus ensuring a proper and functional artificial blood vessel. These implantable devices are sutured and may be used to replace part or entirely coronary arteries. The tubes produced by the present method may also found applications in the field of neural and spine surgery as guides for nerves. They may also be used for treating incontinence as substitutes of urethra and anal conduits. Devices for reconstruction of larynx and oesophagi following oncology surgical or radiotherapy treatments may also be produced by using the disclosed method and device.

One of a particular advantage of the devices composed of nanometer sized polymer fibers is that cells attach, proliferate and differentiate onto these biomimetic structures. Cells produce an abundant extracellular matrix while other cells such as macrophages degrade the nanofibrillar polymer. The synthetic device serves as a scaffold for tissue repair and regeneration and is ultimately replaced by a functional and normal biological tissue.

While the invention has been illustrated and described in details in the drawings and foregoing description, such illustration and description are to be considered illustrative or exemplary and not restrictive; the invention is not limited to the disclosed embodiment.

Other variations to the disclosed embodiments can be understood and effected by those skilled on the art in practicing the claimed invention, from a study of the drawings, the disclosure and the appended claims. In the claims, the word "comprising" does not exclude other elements and the indefinite article "a" or "an" does not exclude a plurality.

## Claims

1. Method for forming nanofiber web comprising the steps of:
- mixing (21) polymeric material with a solvent to obtain a liquid mixture having a viscosity above a predetermined shear viscosity value at room temperature;
- feeding (23) a spray nozzle with said mixture;
- projecting (25) said mixture through said nozzle with a gas jet, wherein the projected polymeric material solidifies and forms nanofibers;
- depositing (27) said nanofibers on a collecting surface to form said nanofiber web.

2. Method according to claim 1, wherein said predetermined shear viscosity value is equal to 0.068 Pa.s for a shear rate increasing from 0.05 to 9000 s⁻¹ in 60s.

3. Method according to claim 2, wherein the shear viscosity of said mixture is above 0.1 Pa.s for a shear rate increasing from 0.05 to 9000 s⁻¹ in 60s.

4. Method according to claim 1, wherein said mixture comprises biological active molecules.

5. Method according to claim 1, wherein said spray nozzle comprises a passageway for directing said mixture outside, said passageway being partially obstructed by an internal needle parallel to the mixture flow, said needle diffracting the mixture outflow.

6. Method according to claim 5, wherein the spray nozzle is of internal mixing type.

7. Method according to claim 5, wherein the spray nozzle is of external mixing type.

8. Method according to claim 7, wherein the gas jet sucks up the mixture outside the spray nozzle.

9. Method according to claim 1, wherein the polymeric material comprises any solvent-soluble polymer, particularly polyester.

10. Device for forming nanofiber web comprising:
- a container (1) of a mixture of a polymeric material and a solvent at room temperature, said mixture having a shear viscosity above a predetermined viscosity value, the container being connected to
- a spray nozzle (7) fed with said mixture;
- a gas jet inlet (17), connected to the spray nozzle so that the gas jet projects said mixture through said nozzle and the projected polymeric material solidifies and forms nanofibers;
- a collecting surface (19) on which said nanofibers deposit to form said nanofiber web.

11. Device according to claim 10, wherein the collecting surface (19) is formed by the surface of a liquid in which the nanofibers are not soluble.

12. Biomimetic nanofiber web produced by the method according to any one of claims 1 to 10 wherein the average mesh size of interlaced polymeric nanofibers is greater than 10 µm and the average diameter of said nanofibers is less than 800 nm.

13. Biomimetic nanofiber web according to claim 12, further comprising biological active molecules interlaced between the polymeric nanofibers.

14. Biomimetic nanofiber web according to claim 13, wherein the biological active molecules are made of calcium phosphate.

## Patentansprüche

1. Verfahren zur Herstellung einer Nanofaserbahn, wobei das Verfahren die folgenden Schritte aufweist:
- Mischen (21) von Polymermaterial mit einem Lösungsmittel, um ein flüssiges Gemisch mit einer Viskosität oberhalb eines vorgegebenen Scherviskositätswerts bei Raumtemperatur zu erhalten;
- Speisen (23) einer Spritzdüse mit dem Gemisch;
- Ausstoßen (25) des Gemischs durch die Düse mit einem Gasstrahl, wobei das ausgestoßene Polymermaterial erstarrt und Nanofasern bildet;
- Ablagern (27) der Nanofasern auf einer Auffangoberfläche, um die Nanofaserbahn zu formen.

2. Verfahren nach Anspruch 1, wobei der vorgegebene Scherviskositätswert für eine in 60 s von 0,05 auf 9000 s⁻¹ ansteigende Scherrate gleich 0,068 Pa·s ist.

3. Verfahren nach Anspruch 2, wobei die Scherviskosität des Gemischs für eine in 60 s von 0,05 auf 9000 s⁻¹ ansteigende Scherrate oberhalb 0,1 Pa·s ist.

4. Verfahren nach Anspruch 1, wobei das Gemisch biologisch aktive Moleküle aufweist.

5. Verfahren nach Anspruch 1, wobei die Spritzdüse einen Durchlass aufweist, um das Gemisch nach außen zu lenken, wobei der Durchlass teilweise durch eine zur Fließrichtung des Gemischs parallele innere Nadel blockiert wird, wobei die Nadel das Ausfließen des Gemischs ablenkt.

6. Verfahren nach Anspruch 5, wobei die Spritzdüse vom Typ mit internem Mischen ist.

7. Verfahren nach Anspruch 5, wobei die Spritzdüse vom Typ mit externem Mischen ist.

8. Verfahren nach Anspruch 7, wobei der Gasstrahl das Gemisch außerhalb der Spritzdüse aufsaugt.

9. Verfahren nach Anspruch 1, wobei das Polymermaterial irgendein durch Lösungsmittel lösliches Polymer, insbesondere einen Polyester aufweist.

10. Vorrichtung zur Herstellung einer Nanofaserbahn, die aufweist:
- einen Behälter (1) für ein Gemisch aus einem Polymermaterial und einem Lösungsmittel bei Raumtemperatur, wobei das Gemisch eine Scherviskosität oberhalb eines vorgegebenen Viskositätswerts aufweist, wobei der Behälter mit den folgenden Komponenten verbunden ist:
- einer Spritzdüse (7), die mit dem Gemisch gespeist wird;
- einem Gasstrahleinlass (17), der mit der Spritzdüse so verbunden ist, dass der Gasstrahl das Gemisch durch die Düse ausstößt und das ausgestoßene Polymermaterial erstarrt und Nanofasern bildet;
- einer Auffangoberfläche (19), auf der sich die Nanofasern ablagern, um die Nanofaserbahn zu formen.

11. Vorrichtung nach Anspruch 10, wobei die Auffangoberfläche (19) durch die Oberfläche einer Flüssigkeit gebildet wird, in der die Nanofasern nicht löslich sind.

12. Biomimetische Nanofaserbahn, die durch das Verfahren nach einem der Ansprüche 1 bis 10 produziert wird, wobei die mittlere Maschenweite von verkreuzten Nanofasern größer als 10 µm ist und der mittlere Durchmesser der Nanofasern kleiner als 800 nm ist.

13. Biomimetische Nanofaserbahn nach Anspruch 12, die ferner biologisch aktive Moleküle aufweist, die zwischen den polymeren Nanofasern verkreuzten sind.

14. Biomimetische Nanofaserbahn nach Anspruch 13, wobei die biologisch aktiven Moleküle aus Calciumphosphat bestehen.

## Revendications

1. Procédé de formation d'une nappe de nanofibres comprenant les étapes de :
- mélange (21) d'un matériau polymère avec un solvant pour donner un mélange liquide ayant une viscosité supérieure à une valeur de viscosité de cisaillement prédéterminée à la température ambiante ;
- alimentation (23) d'une buse de pulvérisation avec ledit mélange ;
- projection (25) dudit mélange à travers ladite buse avec un jet de gaz, où le matériau polymère projeté se solidifie et forme des nanofibres ;
- dépôt (27) desdites nanofibres sur une surface collectrice pour former ladite nappe de nanofibres.

2. Procédé selon la revendication 1, dans lequel ladite valeur de viscosité de cisaillement prédéterminée est égale à 0,068 Pa.s pour un taux de cisaillement croissant de 0,05 à 9000 s⁻¹ en 60 s.

3. Procédé selon la revendication 2, dans lequel la viscosité de cisaillement dudit mélange est supérieure à 0,1 Pa.s pour un taux de cisaillement croissant de 0,05 à 9000 s⁻¹ en 60 s.

4. Procédé selon la revendication 1, dans lequel ledit mélange comprend des molécules biologiques actives.

5. Procédé selon la revendication 1, dans lequel ladite buse de pulvérisation comprend un passage pour diriger ledit mélange vers l'extérieur, ledit passage étant partiellement obstrué par une aiguille intérieure parallèle au flux du mélange, ladite aiguille diffractant le flux de mélange sortant.

6. Procédé selon la revendication 5, dans lequel la buse de pulvérisation est du type mélange interne.

7. Procédé selon la revendication 5, dans lequel la buse de pulvérisation est du type mélange externe.

8. Procédé selon la revendication 7, dans lequel le jet de gaz aspire le mélange à l'extérieur de la buse de pulvérisation.

9. Procédé selon la revendication 1, dans lequel le matériau polymère comprend tout polymère soluble dans un solvant, en particulier un polyester.

10. Dispositif de formation d'une nappe de nanofibres comprenant :
- un récipient (1) d'un mélange d'un matériau polymère et d'un solvant à la température ambiante, ledit mélange ayant une viscosité de cisaillement supérieure à une valeur de viscosité prédéterminée, le récipient étant relié à :
- une buse de pulvérisation (7) alimentée en ledit mélange ;
- une entrée de jet de gaz (17) reliée à la buse de pulvérisation, de sorte que le jet de gaz projette ledit mélange à travers ladite buse et le matériau polymère projeté se solidifie et forme des nanofibres ;
- une surface collectrice (19) sur laquelle lesdites nanofibres se déposent pour former ladite nappe de nanofibres.

11. Dispositif selon la revendication 10, dans lequel la surface collectrice (19) est formée par la surface d'un liquide dans lequel les nanofibres ne sont pas solubles.

12. Nappe de nanofibres biométriques produite par le procédé selon l'une quelconque des revendications 1 à 10, dans laquelle la taille de maille moyenne des nanofibres polymères entrelacées est supérieure à 10 µm et le diamètre moyen desdites nanofibres est inférieur à 800 nm.

13. Nappe de nanofibres biométriques selon la revendication 12, comprenant en outre des molécules biologiques actives entrelacées entre les nanofibres polymères.

14. Nappe de nanofibres biométriques selon la revendication 13, dans laquelle les molécules biologiques actives sont faites de phosphate de calcium.
